(19) Europäisches Patentamt
European Patent Office
Office européen des brevets

(11) Veröffentlichungsnummer: **0 192 674**
**B1**

(12) # EUROPÄISCHE PATENTSCHRIFT

(45) Veröffentlichungstag der Patentschrift: **24.10.90**

(51) Int. Cl.[5]: **A 61 M 1/10**

(21) Anmeldenummer: **85904104.8**

(22) Anmeldetag: **06.08.85**

(86) Internationale Anmeldenummer:
**PCT/EP85/00396**

(87) Internationale Veröffentlichungsnummer:
**WO 86/01116 27.02.86 Gazette 86/05**

(54) **AUTOTRANSFUSIONSGERÄT.**

(30) Priorität: **09.08.84 DE 3429298**

(43) Veröffentlichungstag der Anmeldung:
**03.09.86 Patentblatt 86/36**

(45) Bekanntmachung des Hinweises auf die Patenterteilung:
**24.10.90 Patentblatt 90/43**

(84) Benannte Vertragsstaaten:
**AT BE CH FR GB IT LI LU NL SE**

(56) Entgegenhaltungen:
**EP-A-0 072 738**
**EP-A-0 094 682**
**EP-A-0 116 352**
**FR-A-2 182 578**
**FR-A-2 346 238**
**GB-A-2 016 408**
**US-A-4 041 944**

(73) Patentinhaber: **Rühland, Dieter, Prof. Dr. med.**
**Jungeblodtplatz 1**
**D-4400 Münster (DE)**

(72) Erfinder: **Rühland, Dieter, Prof. Dr. med.**
**Jungeblodtplatz 1**
**D-4400 Münster (DE)**

(74) Vertreter: **Schumacher, Horst, Dr. Dipl.-Phys. et al**
**Patentanwälte Dipl.-Phys. Dr. Peter Palgen Dipl.-Phys. Dr. H. Schumacher Frühlingstrasse 43A (Ecke Holunderweg)**
**D-4300 Essen 1 (DE)**

**EP 0 192 674 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein Autotransfusiongerät für Blut oder der gleichen Körperflüssigkeit sowie ein Verfahren zum Betreiben eines Autotransfusionsgerätes gemäß den Oberbegriffen der Ansprüche 1 bzw. 22.

Bei Operationen, z.B. aus dem Bereich der Herzchirurgie, Gefäbchirurgie, Unfallchirurgie oder Orthopädie kommt es häufig zu großen schnellen Blutverlusten, die heute zumeist noch durch Fremdblut ausgeglichen werden. Fremdblut ist jedoch geeignet, Krankheiten zu übertragen (z.B. Hepatitis). Weiterhin fehlendem oft mehrere Wochen alten Fremdblut die Gerrinnungselemente (Gerinnungsfaktoren und Blutplättchen), die durch Lagerung zerstört werden. Es ist deshalb dringend erforderlich, Geräte zur Verfügung zu haben, die geeignet sind, das sich in den Körperhöhlen während einer Operation ansiedelnde Blut zurückzugewinnen und dem Patienten wieder zuzuführen, um den Fremdbluteinsatz zu verringern und die Blutgerinnungselemente weitgehend zu erhalten.

Es stehen zur Rückgewinnung von Eigenblut (Autotransfusion) für große Herzoperationen komplizierte, teure Herz-Lungen-Maschinen heute zur Verfügung, die das Blut des Patienten über einen Pumpmechanismus absaugen und über lange Schläuche wieder zuführen. Andere Geräte sammeln die Blutflüssigkeit, isolieren und waschen die roten Blutzellen und führen diese gewaschenen Blutzellen dem Körper wieder zu. Bei beiden Geräten werden die empfindlichen Blutzellen durch lange Saugwege geschädigt (Oberflächenkontakt) und bei dem zweiten Gerät werden durch den Waschvorgang alle Gerinnungselemente und das Blutplasma entfernt. Diese Geräte sind zudem sehr teuer und daher nur in wenigen Zentren verfügbar.

Es sind daher weitere Geräte für die intraoperative Autotransfusion entwickelt worden. So ist aus der US—PS 4 047 526 ein Autotransfusionsgerät der eingangs genannten Art bekannt, bei dem das Blut zunächst in einem harten Behälter durch Unterdruck gesammelt und anschließend in einen mit dem harten Sammelbehälter bodenseitig lösbar verbundenen Faltbalg, mittels dessen ein Vakuum erzeugbar ist, durch welchen das Vakuum in dem darüberliegenden harten Behälter überwunden werden kann, überführt. Der mit Blut gefüllte Faltbalg wird dann von dem harten (formsteifen) Behälter gelöst und anschließend zur aktiven Rücktransfusion des Blutes verwendet.

In der US—PS 4 033 345 wird eine andere Ausführungsform eines Autotransfusionsgerätes mit einem formsteifen Zweikammersystem beschrieben, das einen innenliegenden verformbaren Beutel enthält, der mit der oberen Kammer, die zunächst das Blut sammelt, durch ein Rückschlagventil verbunden ist. Das Blut kann durch eine weitere Öffnung mit Rückschlagventil und Filtereinheiten durch Eindringen eines Druckmediums in den Raum zwischen der harten, zweiten Kammer und der äußeren Beuteloberfläche aktiv in den Patienten zurücktransfundiert werden. Die Überleitung des Blutes aus dem formsteifen Sammelbehälter in den flexiblen Beutel erfolgt durch wechselseitiges Anlegen von Über- und Unterdruck.

Bei einem aus der nachveröffentlichen DE—OS 32 18 561 bekannten, weiteren Autotransfusionsgerät handelt es sich um eine der zweiten Kammer der vorerwähnten US—PS 4 033 345 entsprechende Vorrichtung, mit der das Blut jedoch ohne Rückschlagventile durch eine Öffnung in der Kammerunterseite ausgesaugt werden muß und bei der weder ein Sieb noch ein ähnlicher Slutfilter einsetzbar ist.

Schließlich ist aus der US—PS 4 014 329 ein weiteres Zweikammerautotransfusionsgerät bekannt, bei dem die erste Kammer nach dem gleichen Prinzip arbeitet, wie es in der US—PS 4 033 345 für die zweite Kammer beschrieben wurde, wobei hier aber das Blut durch seine Schwerkraft an der Kammerunterseite in die zweite Kammer mit Filter ablfießt.

Mit diesen bekannten Autotransfusionsgeräten ist es zwar möglich, intraoperativ anfallendes Blut zu sammeln und in den Patienten zu retransfundieren, doch weisen sie eine Reihe entscheidender Nachteile auf: Bei den Mehrkammersystemen kommt das Blut mit einer großen Geräteoberfläche in Kontakt, was eine nachteilige Gerinnungsaktivierung und Bluttraumatisierung bewirkt. Ebenso erfolgt eine für die empfindlichen Blutzellen nachteilige, weitere Traumatisierung bei der Überführung von der einen in die andere Kammer, insbesondere wenn zwischen den Kammern Rückschlagventile eingesetzt sind.

Soweit das Blut von unten in die Unterdruckkammer eingesaugt wird, wird das im Gerät bereits angesammelte Blut durch nachfolgend angesaugtes Blut in turbulente Bewegung versetzt, wobei mitangesaugte Luft und grobe Bestandteile, wie Blut, Koagel, Fettzellen und Knochensplitter, eine erhebliche Schaumbildung sowie Traumatisierung der Blutzellen bewirken. Beim Fehlen von Grobfiltern zum Zurückhalten von im Blut mitgeführten Stoffen besteht schließlich bei den herkömmlich an den Transfusionsbestecken vorgesehenen Feinfiltern eine latente Verstopfungsgefahr. Weiterhin erfordern die dekkel- sowie bodenseitig mit Ein- bzw. Auslauföffnungen versehenen Blutsammelkammern eine im Hinblick auf das erforderliche Blutsauglumen beachtliche Bauhöhe, welche regelmäßig höher als das sehr kleine sterile Operations-Gebiet am Patienten ist; daher ergeben sich bei den bekannten Autotransfusionsgeräten regelmäßig Sterilitätsprobleme im sterilen Operations-Gebiet, die nur dadurch ausgleichbar sind, daß das Autotransfusionsgerät außerhlab des Sterilbereiches aufgestellt und ein längerer Saugweg in Kauf genommen wird. Hierdurch ist ein höherer Saugdruck erforderlich und ein vermehrter Fremdkörperkontakt gegeben — beides bedeutet ein zusätzliches Trauma für das Blut. Bei Zweikammersystemen erfordert die Überleitung des Blutes nach der Aspiration in die zweite Kammer Zeit, die weder dem Anästhesisten, noch dem Chirurgen bei einem Massenanfall von Blut zur Verfügung steht: die Zeitfaktor ist besonders aber auch für den Patienten nachteilig, da er das Blut in diesem Falle besonders schnell wieder benötigt.

Aus der FR—A—23 46 238 ist eine Blutpumpe bekannt, deren Aufgabe darin besteht, eine Blut-/Luftoberfläche bei der Blutaufnahme zu vermeiden. Zu diesem Zweck wird der Über- und Unterdruck zur Pumpenbetätigung ausschließlich auf eine der Blutsammelkammer bezüglich einer Membran gegeüberliegende Luftkammer ausgeübt, wobei sich die Einlauf- und Auslauföffnung für das Blut im Bodenteil des Pumpenbehälters befinden, während sich die Luftkammer ständig im Deckelteil befindet. Selbst wenn zusammen mit dem Blut versehentlich einmal etwas Luft angesaugt wird, wird diese im Normalfall nicht weiter transportiert und bildet auch keine, das nachströmende Blut schädigende, Blut-/Luftoberfläche, weil sich die Luft zwingendermaßen im höchsten Bereich des Bodenteiles sammelt und das weiter ein- und ausströmende Blut unterhalb dieser Oberfläche angegsaugt und ausgestoßen wird, mit der Luft also nicht in Kontakt kommt. Zwischen der Zulaufleitung und der Ablaufleitung der unteren Kammer ist also eine permanente Flüssigkeitsverbindung gewährleistet. Bei einer derartigen Blutpumpe ist darauf zu achten, daß Strömungswiderstande, wie sie zum Beispiel ein Sieb in der Auslaufleitung sowie davor etwa sich ansammelnde Partikel darstellen würden, vermieden werden. Als Autotransfusionsgerät ist eine derartige Blutpumpe keinesfalls geeignet, weil mit dem Blut angesaugte Luft sowie Fette oder andere Partikel beim Reinfundieren in den Patienten nicht automatisch zurückgehalten werden können.

Aus der EP—A1—00 72 738 ist ein Autotransfusionsgerät mit einem Ansaugstutzen zur Verbindung mit einer Vakuumpumpe bekannt, wobei dieser Stutzen mit einem Ventilkörper verschließer ist, um den Übertritt von Blut in die Vakuumpumpe zu verhindern. Durch diesen Ventilkörper kann jedoch nicht verhindert werden, dasß am Ende der Retransfusion in den Patienten Luft aus dem Blutsammelraum in den Patienten zurückgeführt wird.

Die Probleme wurden durch ein Autotransfusionsgerät der eingangs genannten Art gelöst (deutsche Patentanmeldung P 33 04 486.4). Bisher ungelöst blieb aber das Problem der im Autotransfusionsgerät vor der Infusion neben dem Blut vorliegenden Luft, die zur Vermeidung einer Luftembolie zu entfernen ist; das Autotransfusionsgerät darf also nur so lange eine Infusion ermöglichen, wie keine Luft in den Patienten infundiert werden kann.

Davon ausgehend liegt der Erfindung die Aufgabe zugrunde, ein Autotransfusionsgerät der eingangs genannten Art zu schaffen, mit dem es möglich ist, das Blut auf kurzem Wege ohne Schädigung zu sammeln und schnell bei sicherer Funktionstüchtigkeit und ohne Schädigungsmöglichkeit für den Patienten, insbesondere ohne die Gefahr einer Luftembolie, in diesen zurückzutransfundieren; insbesondere sollen die vom Blut berührten Kontaktflächen möglichst klein sein und Engstellen sowie Schaumbildung vermieden werden und eine geringe Bauhöhe erreicht werden, die eine Anwendung im Sterilbereich des Patienten gestattet.

Als technische Lösung, wird dafür vorgeschlagen, daß ein Autotransfusionsgerät mit den Merkmalen des Anspruchs 1 und ein Verfarhen mit den Merkmalen des Anspruchs 22 vorgeschlagen.

Die Erfindung beruht demnach auf dem Grundgedanken, im Deckelteil einer einzigen Blutsammelkammer sowohl die Bluteinlaßöffnung, als auch die Blutauslaßöffnung mit vorgeschaltetem Grobfilter und weiterhin vorgeschaltetem Abdicht-Schwimmkörper anzuordnen, so daß das angesaugte Blut von oben in diese Kammer einströmt und ohne Schaumbildung an der Kammerwandung entlang nach unten fließt; bei der Retransfusion wird das Gerät auf den Kopf gestellt und das Blut fließt, gefiltert, durch Schwerkraft und/oder erhöhten Gasdruck in dem durch eine Membran von der Blutsammelkammer abgetrennten Bodenraum zurück zum Patienten, wobei der Abdicht-Schwimmkörper die Auslauföffnung automatisch verschließt, sobald alles Blut ausgelaufen ist; die vorangehende Blutansaugung wird dabei durch einen Unterdruck bewirkt, der entweder — bei gasdicht verschlossenem Bodenraum — an der Auslauföffnung, oder — bei gasdicht verschlossener Auslauföffnung — im Bodenraum angelegt wird; die Membran ist demnach nur an ihrem einzigen Rand zwischen Boden und Deckelteil festgelegt, wobei das Größenverhältnis zwischen Boden- und Dekkelteil unerheblich ist und lediglich gewährleistet sein muß, daß sich die Membran alternierend sowohl an der Bodenteilals auch an der Deckelteilwandung möglichst weitgehend anlegen kann, so daß abwechselnd der Boden und der Deckelraum ein minimales Restvolumen besitzen.

Die Einlauf- und Auslauföffnungen müssen bei unterdruckanwendung im Deckelraum hinreichend weit beabstandet sein, um einen Blutübertritte in die angelegte Unterdruckquelle zu vermeiden.

Das erfindungsgemäße Autotransfusionsgerät weist eine Reihe wichtiger Vorteile auf: Das Blut wird in einer einzigen Kammer ohne verengende Querschnitte, ohne Schaumbildung unter kontrolliert erzeugbarem Druck gesammelt und vorgefiltert aus derselben Kammer unter Anwendung eines frei vorgebbaren Druckes mit der gewünschten Schnelligkeit direkt in den Patienten infundiert, ohne daß am Ende der Infusion etwa neben dem Blut vorhandene Luft in Gefäße des Patienten gelangen kann; hierfür ist nur eine sehr geringe Bauhöhe des unterdruckfesten Behälters erforderlich, so daß dieser jederzeit im Sterilbereich der Operation handhabbar ist. Zudem werden Dichtigkeitsprobleme im Bereich der drei Behälteröffnungen und im Zusammenhang mit der Membran durch vermieden, daß diese Öffnungen allesamt in der im wesentlichen starren Wandung des Deckel- bzw. Bodenteiles und daher unabhängig vom Dichtungsrand der Membran, angeordnet sind; es braucht also lediglich der Membranrand in der funktionell dafür am besten geeigneten Weise zwischen Deckel- und Bodenteil festgelegt werden, während Durchlaßöffnungen mit Schlauchanschlüssen innerhalb der Membranfläche entfallen; hierdurch können die Membraneigenschaften und die Membranabdichtung besonders funktionsgerecht und unabhängig von der Ein- und Auslaßöffnung frei ausgewäghlt werden.

Zweckmäßige Ausgestaltungen des Erfindungsgegenstandes, die insbesondere eine gute Dichtwirkung des Schwimmkörpers sowie eine einfache Handhabung des Autotransfusionsgerätes gewährleisten und Schädigungen des Blutes sowie des Patienten bestmöglich vermeiden, sind in weiteren Ansprüchen enthalten.

Weitare Einzelheiten, Merkmale und Vorteile des Gegenstandes der Erfindung ergeben sich aus der nachfolgenden Beschreibung der zugehörigen Zeichnung, in der eine bevorzugte Ausführungsform eines erfindungsgemäßen Autotransfusionsgerätes dargestellt worden ist. In der Zeichnung zeigen:

Fig. 1 ein Autotransfusionsgerät im Längsschnitt;

Fig. 2 ein anderes Autotransfusionsgerät im Längsschnitt — ausschnittsweise (Deckelteil);

Fig. 3 ein anderes Autotransfusionsgerät im Längsschnitt — ausschnittsweise (Deckelteil) sowie

Fig. 4 ein anderes Autotransfusionsgerät im Längsschnitt — ausschnittsweise (Deckelteil).

Fig. 1 zeigt den wesentlichen Teil eines Autotransfusionsgerätes in Form eines im ganzen mit 5 bezeichneten, aus sterilizierbarem Kunstoff oder Glas bestehenden Behälter, der als Einmal-Behälter ausgeführt sein kann. Der Behälter weist einen zylinderischen oder rechtwinkligen Querschnitt auf und besteht aus einem Bodenteil 4 und einem mit dessen oberem Rand fluiddicht verbundenen Deckelteil 3. Der Behälter 5 ist unterdruckstabil und über eine Auslauföffnung 24 im Deckelteil 3 oder eine Öffnung 9 im Bodenteil 4 evakuierbar. Im Deckelteil 3 ist in ausreichendem Abstand von der Auslauföffnung 24 eine Einlauföffnung 20 im Bereich eines trompetenförmigen Mundstücks 29 tangential zur Innenwand 21 des Deckelteils 3 angeordnet, so daß vom Patienten angesaugte Körperflüssigkeit an dieser Innenwand durch Gravitation ohne Schaumbildung hinabläuft. Eine Sieb 25 zum Zurückhalten vom im Blut mitgeführten Grobstoffen deckt die Auslauföffnung 24 mit Abstand vom Behälterinnenraum her ab. Die Öffnung 9 ist gasdicht verschließbar und ermöglicht nach Entfernen des Verschlusses das Eindringen atomsphärischer Luft oder von Druckluft in das Behälterinnere. Eine durch Druck verformbare, für die Körperflüssigkeit undurchlässige Membran 16 ist zwischen dem Bodenteil 4 und dem Deckelteil 3 randseitig gasdicht derart festgelegt, daß sie den Behälter 5 in einen gasgefüllten Bodenraum 13 und einen demgegenüber abgedichteten Dekkelraum 19 — zum Aufnehmen der Körperflüssigkeit — unterteilt. Unter dem Einfluß eines Fluiddruckes kann sich die Membran 16, wie gestrichelt dargestellt, an die Innenkontur 1 des Deckelteils 3 sowie alternierend and die Innenkontur 2 des Bodenteils 4 im wesentlichen anlegen. Selbstverständlich sind alle Zwischenpositionen der Membran 16 zwischen diesen beiden Extrempositionen einnehmbar, was durch weitere gestrichelte Linien und die Richtungspfeile B und C dargestellt ist.

Die Verbindung zwischen dem Membranrand und dem Boden- und Deckelteil kann durch Kunststoffverschweißen unter Bildung eines einteiligen Einmal-Behälters erfolgen.

Bei völlig mit Blut gefüllten Behältern nimmt die Membran 16 im wesentlichen die Form der Innenkontur 2 ein. Dann wird die Zulaufleitung vom Patienten dicht verschlossen und ggf. patientenseitig abgeklemmt. Nach dem Entfernen einer nicht dargestellten Vakuumleitung, die entweder an die Auslaßöffnung 24 oder an die Öffnung 9 angeschlossen ist, kann durch Einlassen von Druckluft durch die Öffnung 9 und Anschliessen eines Retransfusionsbesteckes an die Öffnung 24 die im Dekkelraum 3 und den nachgeordneten Systembereichen noch vorhandene Luft ausgetrieben werden. Dabei hält das Sieb 25 Grobbestandtiel zurück. Nach dem Auf-den-Kopf-Drehen des Behälters 5 haben sich nach einer Beruhigungszeit alle Leichtstoffe des Blutes, wie noch nicht ausgetriebene Luftbläschen und Fettzellen, unter der — dann oben liegenden — Membran 16 gesammelt. Beim anschließenden Einlassen von Umgebungs — oder Druckluft durch die Öffnung 9 strömt das Blut so lange zum Patienten zurück, bis die Membran 16 sich an die Kontur 1 des Deckelteils 3 sowie an das Sieb 25 ganz angelegt hat. Dabei verbleiben zwischen Membran 16 und Sieb 25 im Deckelraum 19 nur noch vom Sieb zurückgehaltene Grobbestandteile, während Feinbestandteile, wie die erwähnten Luftblasen oder Fettzellen dann konzentriert im Raum 26 zwischen der Auslauföffnung 24 und dem Sieb 25 angesammelt sind und nicht mehr zum Patienten gelangen können.

Ein Schwimmkörper 7 kann als Kugel ausgebildet und zwischen der Auslauföffnung 24 und dem Sieb 25 angeordnet sein. Die Kugeloberfläche bildet dabei die schwimmkörperseitige Dichtfläche 10 und die die Auslauföffung 24 umgebende Innenfläche des Deckelteils 3 bildet die behälterseitige Dichtfläche 8 für den Schwimmkörper. Die Dichtfläche 8 ist bevorzugt konisch ausgeführt und konvergiert in Richtung der Auslauföffnung 24.

Der Schwimmkörper 7 soll eine ausreichend hohen Auftrieb in der zu transfundierenden Körperflüssigkeit aufweisen, so daß er nicht etwa durch Adhäsion oder dgl. an den ihn umgebenden Bauteilen des Behälters 5 haften bleibt, wenn der Flüssigkeitspiegel steigt oder fällt. Der Schwimmkörper 7 soll also so viel Auftrieb haben, daß er stets auf der Körperflüssigkeit aufschwimmt, d.h. z.T. in sie eingetaucht und z.T. aus ihr ausgetaucht ist. Dies kann vor allem mit innen hohlen Schwimmkörpern erreicht werden. Der Auftrieb des Schwimmkörpers soll andererseits nicht so groß sein, daß der Schwimmkörper beim Evakuieren des Deckelsraumes 19 durch den Evakuiergasstrom derart angehoben werden kann, daß der Evakuiergasstrom dadurch unterbrochen wird. Im übrigen soll zumindest die Oberfläche des Schwimmkörpers aus einem blutfreundlichen Material, wie PTFE, Polyurethan oder einem Silikon bestehen; z.B. kann ein geeignet geformtes Korkstück oder dgl. für den Auftrieb des Schwimmkörpers sorgen, während ein Überzug aus dem blutfreundlichen Material die Dichtfläche des Schwimmkörpers bildet.

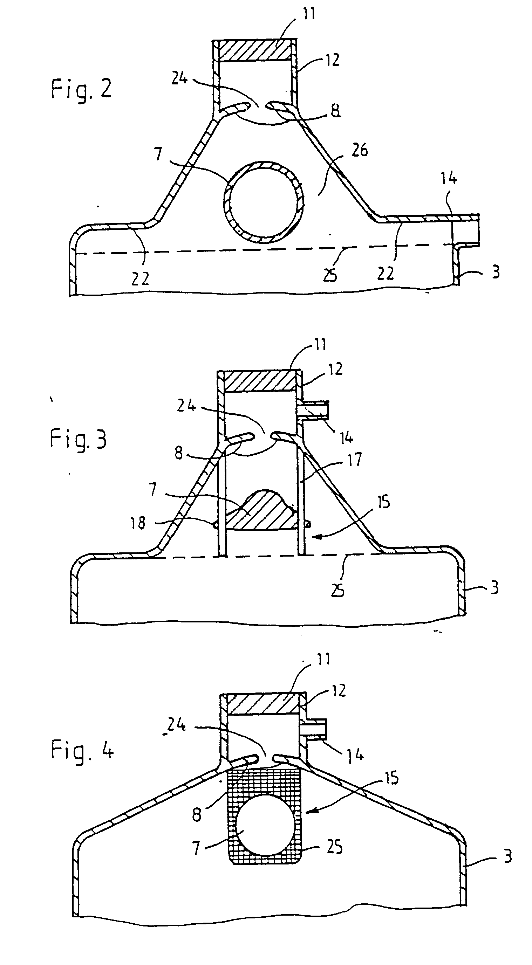

Bei den Ausführungsformen des Deckelteils 3 eines erfindungsgemäßen Autotransfusionsgerätes gemäß den Fig. 2 bis 4 kann der Auslauföffnung 24 ein mit einer Durchstechmembran 11 verschlossener Blutauslauf 12 nachgeordnet sein. Solche Durchstechmembranen für Injektionsnadeln und dgl. sind in der medizinischen Anwendungstecknik weit verbreitet und brauchen deshalb nicht näher beschrieben zu werden.

Gemäß den Fig. 3 und 4 kann der Auslauföffnung 24 ein von dem Blutauslauf 12 unabhängiger Evakuieranschluß 14 nachgeordnet sein. Dies hat den Vorteil, daß beim Einsaugen der Körperflüssigkeit diese nicht in die Vakuumpumpe gelangen kann, well der Schwimmkörper 7 auf der bereits eingesaugten Körperflüssigkeit aufschwimmt und die Auslauföffnung 24 verschließt, sobald der Deckelraum 19 und der oberhalb des Siebes 25 sich befindende Raum — also der gesamte Behälter 5 — mit Körperflüssigkeit gefüllt ist.

Weiterhin kann gemäß den Fig. 3 und 4 die Auslauföffnung 24 von einem Schwimmkörpereinfangkäfig 15 umgeben sein. Dieser dient der sicheren Führung des Schwimmkörpers kurz bevor er die Dichtfläche 8 an der Auslauföffnung 24 erreicht. Als Schwimmkörpereinfangkäfig sind z.B. Vorsprünge an der Deckelinnenseite geeignet, deren schwimmkörperseitige Stirnkanten den Schwimmkörper in Richtung auf die Auslauföffnung 24 führen können. Gemäß Fig. 3 kann ein Schwimmkörpereinfangkäfig auch aus mehreren Stäben 17 bestehen, die sich zwischen der Innenfläche des Deckelteils und dem Sieb 25 erstrekken. Insbesondere kann der Schwimmkörper 7 (gemäß Fig. 3) randseitige Führungselemente 18, wie Ösen, Nuten oder dgl., aufweisen, die mit den Stäben 17 korrespondieren und eine leichte gleitende Führung des Schwimmkörpers 7 ermöglichen. Diese Führungsart ist vor allem für von der Kugelform abweichende Schwimmkörper — wie in Fig. 3 dargestellt — geeignet. Der Schwimmkörpereinfangkäfig 15 kann aber auch durch entsprechende Formgebung und Anordnung des Siebes 25 — gemäß Fig. 4 — von dem Sieb 25 selbst gebildet werden.

Gemäß Fig. 2 kann zwischen dem Sieb 25 und der Auslauföffnung 24 ein Absatz 22 im Deckelteil 3 vorgesehen sein. Hierdurch erhält das Sieb 25 eine möglichst große wirksame Fläche, ohne daß der darüberliegende Raum 26 unnötig groß wird. Es ist nämlich für die ordnungsgemäße Dichtfunktion des Schwimmkörpers 7 wünschenswert, daß die zur Auslauföffnung 24 führenden Seitenwände des Raumes 26 im Deckelteil 3 ausreichend steil sind, um die Führung des Schwimmkörpers 7 zu verbessern. Im Bereich dieses Absatzes 22 kann auch der Evakuieranschluß 14 vorgesehen sein. Ein Verschließen dieses Evakuieranschlusses 14 durch den Schwimmkörpers 7, der in Fig. 2 als Hohlkugel ausgebildet ist, wird durch entsprechende Dimensionierung des Abstandes zwischen dem Sieb 25 und dem Absatz 22 verhindert. Dieser Abstand soll kleiner als der Schwimmkörperdurchmesser sein.

Als besonders vorteilhafte Außenkontur des Schwimmkörpers 7 hat sich die Kugelform erwiesen. Gleichwohl kann im Einzelfall auch eine abgeplattete Kugel oder ein scheibenförmiger Schwimmkörper, ggf. mit einem besonderen Dichtflächenansatz — wie in Fig. 3 — von Vorteil sein.

**Patentansprüche**

1. Autotransfusionsgerät für Blut oder der gleichen Körperflüssigkeit, bestehend aus

a) einem evakuierbaren, unterdruckstabilen, ein Bodenteil (4) und ein Deckelteil (3) aufweisenden Behälter (5) mit

a1) einer im Deckelteil angeordneten Einlauföffnung (20) für die Körperflüssigkeit,

a2) einer im Deckelteil angeordneten Auslauföffnung (24) für die Körperflüssigkeit mit einem die Auslauföffnung abdeckenden Sieb (25) sowie

a3) einer im Bodenteil angeordneten Öffnung (9) zum Herstellen einer Gasströmungsverbindung zu einem Raum mit einem vom Behälterinneren verschiedenen Gasdruck,

b) einer durch Druck verformbaren, gegebenenfalls beutel- oder ballonförmigen fur die Körperflussigkeit undurchlässigen Membran (16), die zwischen dem Boden- und dem Deckelteil randseitig gasdicht festgelegt ist und den Behälter (5) in einen gasgefüllten Bodenraum (13) und einen demgegenüber abgedichteten Deckelraum (19) zum Aufnehmen der Körperflussigkeit unterteilt, dadurch gekennezeichnet, daß

c) die Auslauföffnung (24) durch einen Schwimmkörper (7) abdichtbar ist.

2. Gerät nach Anspruch 1, dadurch gekennzeichnet, daß der Schwimmkörper (7) zwischen der Auslauföffnung (24) und dem Sieb (25) angeordnet ist.

3. Gerät nach Anspruch 1 oder 2, dadurch gekennzeichnet, daß die Auslauföffnung (24) an der Deckelinnenseite von einer kegeligen Dichtfläche (8) umgeben ist.

4. Gerät nach einem der Ansprüche 1 bis 3, dadurch gekennzeichnet, daß der Schwimmkörper (7) eine kugelförmige Dichtfläche (10) aufweist.

5. Gerät nach einem der Ansprüche 1 bis 4, dadurch gekennzeichnet, daß der Auslauföffnung (24) ein mit einer Durchstechmembran (11) verschlossener Blutauslauf (12) nachgeordnet ist.

6. Gerät nach einem der Ansprüche 1 bis 5, gekennzeichnet durch einen von dem Blutauslauf unabhängigen, ggf der Auslauföffnung (24) nachgeordnet Evakuieranschluß (14).

7. Gerät nach einem der Ansprüche 1 bis 6, gekennzeichnet durch einen die Auslauföffnung (24) umgebenden Schwimmkörpereinfangkäfig (15).

8. Gerät nach einem der Ansprüche 2 bis 7, gekennzeichnet durch einen zwischen dem Sieb (25) und der Aulauföffnung (24) angeordneten Absatz (22) im Deckelteil (3).

9. Gerät nach einem der Ansprüche 1 bis 8, dadurch gekennzeichnet, daß die Oberfläche des Schwimmkörpers (7) aus einem blutfreundlichen Material besteht.

10. Gerät nach einem der Ansprüche 1 bis 9, dadurch gekennzeichnet, daß der Schwimmkörper (7) die Form einer Kugel aufweist.

11. Gerät nach einem der Ansprüche 1 bis 10, dadurch gekennzeichnet, daß die Membran (16) unter dem Einfluß eines Fluiddruckes sowohl an die Innenkontur (1) des Deckels (3), als auch an die Innenkontur (2) des Bodenteils (4) im wesentlichen anlegbar ist.

12. Gerät nach einem der Ansprüche 1 bis 11, dadurch gekennzeichnet, daß der Behälter (5) mit der Membran (16) als einteiliger Einmal-Behälter, insbesondere aus Kunststoff, ausgebildet ist.

13. Gerät nach einem der Ansprüche 1 bis 12, dadurch gekennzeichnet, daß das Deckelteil (3) lösbar an dem Bodenteil (4) befestigt, insbesondere verschraubt, ist.

14. Gerät nach Anspruch 13, dadurch gekennzeichnet, daß ein Randabschnitt der Membran (16) zwischen Deckel- und Bondenteil eingeklemmt ist.

15. Gerät nach Anspruch 14, dadurch gekennzeichnet, daß ein verstärkter Rand der Membran (16) in einer Ringnut (17) des Deckelrandes gasdicht gehalten ist.

16. Gerät nach einem der Ansprüche 1 bis 15, dadurch gekennzeichnet, daß die Membran (16) ein aufblasbarer, mit seinem Mündungsrand (12) gegenüber dem Rand der Öffnung (9) gasdicht festgelegter Ballon ist.

17. Gerät nach einem der Ansprüche 1 bis 16, dadurch gekennzeichnet, daß die Einlauföffnung (20) tangential zu der Innenwand (21) des Deckelteils (3) mündet.

18. Gerät nach Anspruch 17, dadurch gekennzeichnet, daß die Einlauföffnung (20) aus einem T-förmigen Mundstück besteht.

19. Gerät nach Anspruch 17, dadurch gekennzeichnet, daß die Einlauföffnung (20) aus einem trompetenartig erweiterten Mundstück (29) besteht.

20. Gerät nach einem der Ansprüche 1 bis 19, dadurch gekennzeichnet, daß das Sieb (25) mit Abstand vor der Auslauföffnung (24), insbesondere lösbar, gehalten ist und einen Raum (26) zum Zurückhalten auf der Körperflüssigkeit aufschwimmender, durch das Sieb mitgeführter Stoffe definiert.

21. Gerät nach einem der Ansprüche 1 bis 20, dadurch gekennzeichnet, daß der Schwimmkörper (7) hohl ist.

22. Verfahren zum Betreiben eines Autotransfusionsgerätes für Blut oder der gleichen Körperflüssigkeit, bestehend aus.

a) einem evakuierbaren, unterdruckstabilen, ein Bodenteil und ein Deckelteil aufweisenden Behälter mit

a1) einer im Deckelteil angeordneten Einlauföffnung für die Körperflüssigkeit,

a2) einer im Deckelteil angeordneten Auslauföffnung für die Körperflüssigkeit sowie

a3) einer im Bodenteil angeordneten Öffnung zum Herstellen einer Gasströmungsverbindung zu einem Raum mit einem vom Behälterinneren verschiedenen Gasdruck und

b) einer durch Druck verformbaren, fur die Körperflüssigkeit undurchlässigen Membran, die zwischen dem Boden- und dem Deckelteil randseitig gasdicht festgelegt ist und den Behälter in einen gasgefüllten Bodenraum und einen demgegenüber abgedichteten Deckelraum zum Aufnehmen der Körperflussigkeit unterteilt, gekennzeichnet durch die Verwendung eines die Auslauföffnung nach dem Auf- den-Kopf-Drehen des Behälters mit dem Abschluß des Blutauslaufers abdichtenden Schwimmkörpers.

**Revendications**

1. Appareil d'autotransfusion pour du sang ou un liquide corporel analogue, comportant:

a) un récipient (5) constitué d'une partie fond (4) et d'une partie couvercle (3), et susceptible d'être mis sous vide et de résister à une dépression,

a1) un orifice d'entrée (20) pour le liquide corporel, disposé dans la partie couvercle,

a2) un orifice de sortie (24) pour le liquide corporel, disposé dans la partie couvercle, et comportant un filtre (25),

a3) ainsi qu'un orifice (9), disposé dans la partie fond (4), pour réaliser une liaison d'écoulement gazeux vers un volume comportant une pression gazeuse différente de celle de l'intérieur du récipient,

b) une membrane (16) étanche au liquide corporel, déformable sous l'effet de la pression, le cas échéant en forme de poche ou de ballon, qui est fixée par son bord entre la partie fond et la partie couvercle de façon étanche aux gaz, et qui sépare le récipient (5) en un volume de fond (13), rempli de gaz, et un volume côté couvercle (19), isolé de celui-ci de façon étanche, et destiné à recevoir le liquide corporel, caractérisé en ce que

c) l'orifice de sortie (24) peut être fermé de façon étanche par un corps de flotteur (7).

2. Appareil suivant la revendication 1, caractérisé en ce que le corps de flotteur (7) est disposé entre l'orifice de sortie (24) et le filtre (25).

3. Appareil suivant la revendication 1 ou la revendication 2, caractérisé en ce que l'orifice de sortie (24), du côte de l'intérieur du couvercle, est entouré par une surface d'étanchéité (8) de forme conique.

4. Appareil suivant l'une des revendications 1 à 3, caractérisé en ce que le corps de flotteur (7) présente une surface d'étanchéité (10) de forme sphérique.

5. Appareil suivant l'une des revendications 1 à 4, caractérisé en ce qu'un orifice de sortie du sang (12), fermé avec un membrane perforée (11), est disposé après l'orifice de sortie (24).

6. Appareil suivant l'une des revendications 1 à 5, caractérisé par un raccord de mise sous vide (14) indépendant de l'orifice de sortie du sang, le cas échéant disposé après l'orifice de sortie (24).

7. Appareil suivant l'une des revendications 1 à 6, caractérisé par une cage (15) emprisonnant le corps de flotteur et entourant l'orifice de sortie (24).

8. Appareil suivant l'une des revendications 2 à 7, caractérisé par un rebord (22) dans la partie couvercle (3), situé entre le filtre (25) et l'orifice de sortie (24).

9. Appareil suivant l'une des revendications 1 à 8, caractérisé en ce que la surface externe du corps de flotteur (7) est constituée d'un matériau hématophile.

10. Appareil suivant l'une des revendications 1 à 9, caractérisé en ce que le corps de flotteur (7) présente la forme d'une boule.

11. Appareil suivant l'une des revendications 1 à 10, caractérisé en ce que la membrane (16) peut essentiellement venir s'appuyer, sous l'effet d'une pression de fluide, aussi bien contre le contour interne (1) de la partie couvercle (3), que contre le contour interne (2) de la partie fond (4).

12. Appareil suivant l'une des revendications 1 à 11, caractérisé en ce que le récipient (5) comportant la membrane (16) est réalisé, en particulier en matière plastique, comme un récipient jetable.

13. Appareil suivant l'une des revendications 1 à 12, caractérisé en ce que la partie couvercle (3) est fixée à le partie fond (4), en particulier par des vis, de façon à être détachable.

14. Appareil suivant la revendication 13, caractérisé en ce qu'une bande de bordure de la membrane (16) est pincée entre partie couvercle et partie fond.

15. Appareil suivant la revendication 14, caractérisé en ce qu'un bord renforcé de la membrane (16) est maintenue de façon étanche aux gaz dans une rainure annulaire (17) du bord du couvercle.

16. Appareil suivant l'une des revendications 1 à 15, caractérisé en ce que la membrane (16) est un ballon gonflable, fixé, de façon étanche aux gaz, avec sa lèvre (12) vis-à-vis du bord de l'orifice (9).

17. Appareil suivant l'une des revendications 1 à 16, caractérisé en ce que l'orifice d'entrée (20) débouche tangentiellement à la paroi intérieure (21) de la partie couvercle (3).

18. Appareil suivant la revendication 17, caractérisé en ce que l'orifice d'entrée (20) est constitué d'une embouchure en forme de T.

19. Appareil suivant la revendication 17, caractérisé en ce que l'orifice d'entrée (20) est constitué d'une embouchure s'élargissant en forme de trompette.

20. Appareil suivant l'une des revendications 1 à 19, caractérisé en ce que le filtre 25 est maintenu, en particulier en étant détachable, à une certaine distance de l'orifice de sortie (24) et détermine un volume (26) pour retendir des élémentes surnageant sur le liquide corporel et entraînés par le filtre.

21. Appareil suivant l'une des revendications 1 à 20, caractérisé en ce que le corps de flotteur (7) est creux.

22. Procédé pour mettre en oeuvre l'appareil d'autotransfusion pour du sang ou un liquide corporel analogue, comportant:

a) un récipient constitué d'une partie fond et d'une partie couvercle, et susceptible d'être mis sous vide et de résistant à une dépression,

a1) un orifice d'entrée pour le liquide coporel, disposé dans la partie couvercle,

a2) un orifice de sortie pour le liquide corporel, disposé dans la partie couvercle, et comportant un filtre,

a3) ainsi qu'un orifice, disposé dans la partie fond, pour réaliser une liaison d'écoulement gazeux vers un volume comportant une pression gazeuse différente de celle de l'intérieur du récipient,

b) une membrane étanche au liquide corporel, déformable sous l'effet de la pression, les cas échéant en forme de poche ou de ballon, qui est fixée par son bord entre la partie fond et la partie couvercle de façon étanche aux gaz, et qui sépare le récipient en un volume de fond, rempli de gaz, et un volume côté couvercle, isolé de celui-ci de façon étanche, et destiné à recevoir le liquide corporel, caractérisé par

l'utilisation d'un corps de flotteur assurant l'étanchéité de l'orifice de sortie, aprés que l'on ait retourné tête en bas le récipient, avec fermeture de l'écoulement du sang vers l'extérieur.

## Claims

1. Autotransfusion apparatus for blood or equivalent body fluid, consisting of

a) an evacuable container (5) which is stable under low pressure and which has a base part (4) and a lid part (3), with

a1) an inlet opening (20) for the body fluid, arranged in the lid part,

a2) an outlet opening (24) for the body fluid, arranged in the lid part, with a filter (25) covering the outlet opening, and

a3) an opening (9) arranged in the base part, for producing a gas flow connection to a chamber which has a different gas pressure from that of the interior of the container,

b) a pressure deformable diaphragm (16) optionally in the form of a bag or balloon, and which is impermeable to the body fluid, and which is peripherally secured between the base and lid parts in a gastight manner, and divides the container (5) into a gas-filled base chamber (13) and a lid chamber (19) sealed relative thereto, for receiving the body fluid,
characterised in that

c) the outlet opening (24) is sealable by means of a float member (7).

2. Apparatus according to claim 1, characterised in that the float member (7) is arranged between the outlet opening (24) and the filter (25).

3. Apparatus according to claim 1 or 2, characterised in that the outlet opening (24) is surrounded on the inner side of the lid by a conical sealing surface (8).

4. Apparatus according to any one of the claims 1 to 3, characterised in that the flaot member (7) has a spherical sealing surface (10).

5. Apparatus according to any one of the claims 1 to 4, characterised in that subsequent to the outlet opening (24) there is arranged a blood outlet (12) which is closed by means of a perforated diaphragm (11).

6. Apparatus according to any one of the claims 1 to 5, characterised by an evacuating connection (14) which is independent of the blood outlet and which optionally is arranged subsequent to the outlet opening (24).

7. Apparatus according to any one of the claims 1 to 6, characterised by an enclosure cage (15) for the float member, surrounding the outlet opening (24).

8. Apparatus according to any one of the claims 2 to 7, characterised by a shoulder portion (22) in the lid part (3), arranged between the filter (25) and the outlet opening (24).

9. Apparatus according to any one of the claims 1 to 8, characterised in that the surface of the float member (7) consists of a blood-compatible material.

10. Apparatus according to any one of the claims 1 to 9, characterised in that the float member (7) is in the shape of a sphere.

11. Apparatus according to any one of the claims 1 to 10, characterised in that under the influence of a fluid pressure, the diaphragm (16) can be applied substantially not only against the inner contour (1) of the lid (3), but also against the inner contour (2) of the base part (4).

12. Apparatus according to any one of the claims 1 to 11, characterised in that the container (5), with the diaphragm (16), is constructed, especially of plastics material, as an integral, one-use container.

13. Apparatus according to any one of the claims 1 to 12, characterised in that the lid part (3) is detachably secured on the base part (4), especially screwed on.

14. Apparatus according to claim 13, characterised in that an edge section of the diaphragm (16) is clamped between the lid and the base parts.

15. Apparatus according to claim 14, characterised in that a reinforced edge of the diaphragm (16) is held in a gastight manner in an annular groove ([illegible numeral]) of the edge of the lid.

16. Apparatus according to any one of the claims 1 to 15, charactersed in that the diaphragm (16) is an inflatable balloon which is secured in a gastight manner with its orifice edge (12) opposite the edge of the opening (9).

17. Apparatus according to any one of the claims 1 to 16, characterised in that the inlet opening (20) opens tangentially to the inner wall (21) of the lid part (3).

18. Apparatus according to claim 17, characterised in that the inlet opening (20) consists of a T-shaped mouthpiece.

19. Apparatus according to claim 17, characterised in that the inlet opening (20) consists of a trumpet-shaped widened mouthpiece (29).

20. Apparatus according to any one of the claims 1 to 19, characterised in that the filter (25) is held spaced at a distance before the outlet opening (24), in particular in a detachable manner, and defines a chamber (26) for retaining substances suspended in the body fluid and which are carried out therewith through the filter.

21. Apparatus according to any one of the claims 1 to 20, characterised in that the float member (7) is hollow.

22. Method for operating an autotransfusion apparatus for blood or equivalent body fluid, consisting of

a) an evacuable container which is stable under low pressure and which has a base part and a lid part, with

a1) an inlet opening, for the body fluid, arranged in the lid part,

a2) an outlet opening for the body fluid, arranged in the lid part, and

a3) an opening arranged in the base part, for producing a gas flow connection to a chamber which has a different gas pressure from that of the interior of the container, and

b) a pressure deformable diaphragm which is impermeable to the body fluid and which is peripherally secured in a gastight manner between the base and lid parts, and which divides the container into a gas-filled base chamber and a lid chamber sealed relative thereto, for receiving the body fluid, characterised by the use of a float member which seals the outlet opening after the container has been inverted, when the blood outflow has ceased.

Fig.1

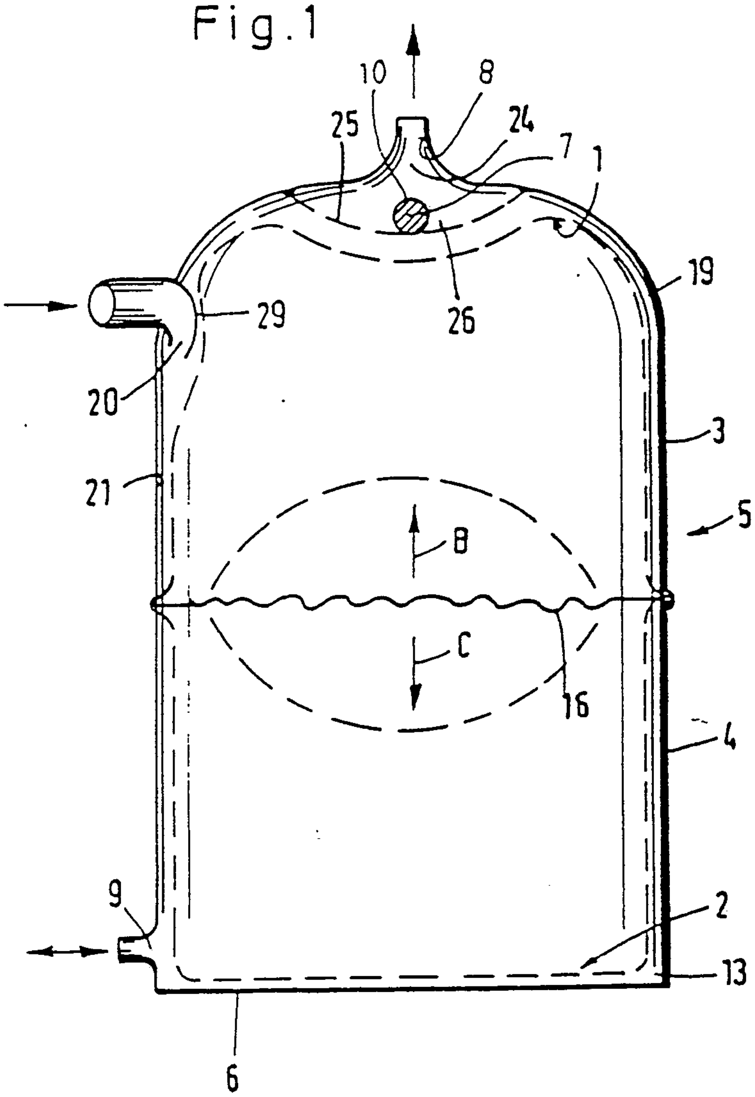

Fig. 2

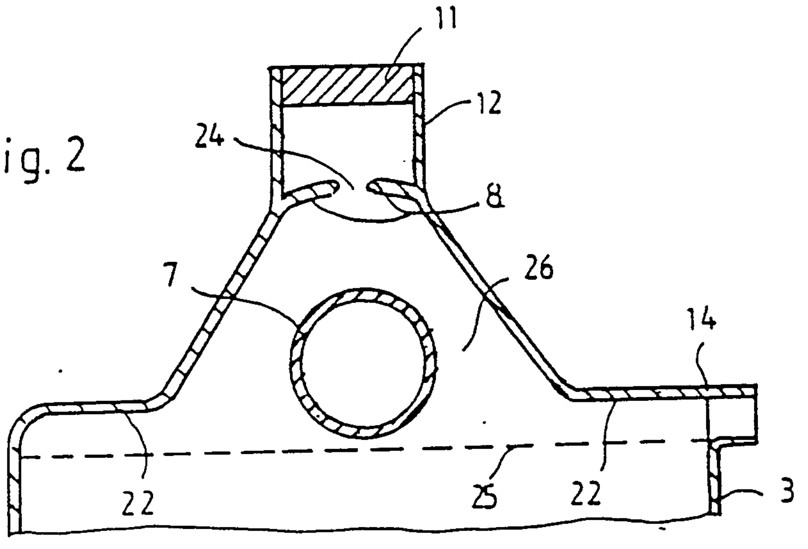


Fig. 3

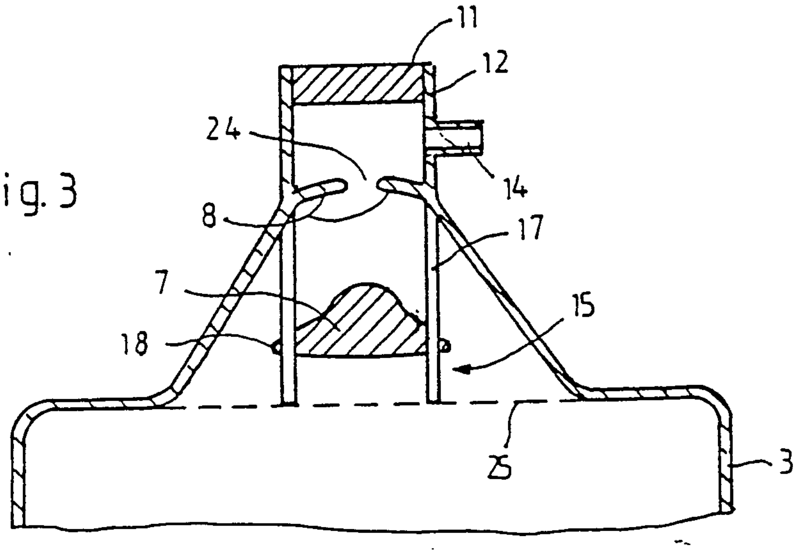


Fig. 4